# EUROPEAN PATENT SPECIFICATION

(11) **EP 2 522 388 B1**
(45) Date of publication and mention of the grant of the patent: **22.06.2016**
(21) Application number: 12167209.1
(22) Date of filing: 09.05.2012
(51) Int. Cl.: A61N 1/36, A61N 1/04, A61B 5/00, A61N 1/05

(54) **Sympathetic skin response measuring apparatus**
Messvorrichtung zur Messung der sympathischen Hautreaktion
Appareil de mesure de la réponse sympathique de la peau

(30) Priority: 10.05.2011 JP 2011105199
(43) Date of publication of application: 14.11.2012
(73) Proprietor: Nihon Kohden Corporation, Shinjuku-ku Tokyo (JP)
(72) Inventor: Motogi, Jun, Tokyo (JP); Ono, Yoshinobu, Tokyo (JP); Ushijima, Ryosuke, Tokyo (JP); Kojima, Takeshi, Tokyo (JP)
(74) Representative: Grünecker Patent- und Rechtsanwälte PartG mbB

(56) References cited:
- EP-A1- 2 174 589
- BHAGWAN T SHAHANI ET AL: "Sympathetic skin response-a method of assessing unmyelinated axon dysfunction in peripheral neuropathies", JOURNAL OF NEUROLOGY, NEUROSURGERY, AND PSYCHIATRY, vol. 47, no. 5, 1 May 1984 (1984-05-01), pages 536-542, XP55035971, DOI: 10.1136/jnnp.47.5.536
- "Surface Stimulation Electrode NM-980W", , 4 August 2010 (2010-08-04), XP55036050, Tokyo, Japan Retrieved from the Internet: URL:http://www.nihonkohden.co.jp/iryo/docu ments/pdf/H901965A.pdf [retrieved on 2012-08-22]
- R. VETRUGNO ET AL: "Sympathetic skin response", CLINICAL AUTONOMIC RESEARCH, vol. 13, no. 4, 1 August 2003 (2003-08-01) , pages 256-270, XP55035878, ISSN: 0959-9851, DOI: 10.1007/s10286-003-0107-5
- PEDRO SCHESTATSKY ET AL: "Skin autonomic reactivity to thermoalgesic stimuli", CLINICAL AUTONOMIC RESEARCH ; AN INTERNATIONAL JOURNAL FOR FAST COMMUNICATIONS OF RESEARCH AND TREATMENT RELATED TO AUTONOMIC FUNCTION AND DYSFUNCTION, STEINKOPFF-VERLAG, DA, vol. 17, no. 6, 29 November 2007 (2007-11-29), pages 349-355, XP019546627, ISSN: 1619-1560, DOI: 10.1007/S10286-007-0446-8

## Description

### BACKGROUND OF THE INVENTION

The present invention relates to a sympathetic skin response measuring apparatus which can adequately perform a sympathetic skin response (SSR) measurement, and more particularly to a sympathetic skin response measuring apparatus which can be suitably applied to screening for diabetes or pain management. However, the invention is not limited to them.

An SSR measurement is widely used as a method of non-invasively testing an autonomic nervous system in various fields such as neurology and psychosomatic medicine. As one of related-art techniques for an SSR measurement, there is a technique which is performed by applying strong electric stimulation or a loud sound to the patient. In a quantitative sensory testing in measurement of neuropathic pain, a technique in which, when the patient feels stimulation, the patient presses a button is employed.

In the technique in which, when the patient feels stimulation, the patient presses a button, in the case where the patient is an aged person, particularly, there is a problem in that a considerable time period is required from perception until the button is pressed, and the measurement cannot be accurately performed.

On the other hand, while using a phenomenon in which, when pain is perceived, sweating occurs in the palm or the like, an SSR measurement in which electrodes are employed is performed. For example, electrodes are disposed on the palm, and stimulation is applied while changing the attitude of the patient from the supine position to the maximum anteflexion position (see JP-A-7-23964). Also there is a measurement in which electrodes are disposed on finger tips and stimulation for determining the type of sleep apnea is applied (see JP-A-2009-82660).

In the above-described measurement in which stimulation is applied while changing the attitude, or in which stimulation for determining the type of sleep apnea is applied, it is impossible to accurately measure the time period from the stimulation timing to the sensing timing, and the normality/abnormality of the sympathetic nerve is hardly known. In the case where electric stimulation is applied, when the stimulation intensity is lowered, there arises a problem in that discrimination from external noises is hardly performed.

In the case where the sympathetic nerve is to be evaluated, particularly, it is said that analysis of C fibers among nerve fibers for skin sensation is important. Namely, it is requested that C fibers are stimulated distinctively from the other nerve fibers.

Bhagwan T Shahani et al. ("Sympathetic skin response-a method of assessing unmyelinated axon dysfunction in peripheral neuropathies",Journal of Neurology, Neurosurgery, and Psychiatry, vol. 47, no. 5, pages 536-542, DOI: 10.1136/ jnnp.47.5.536) disclose a method for evaluating the sympathetic skin response using electrical stimulation.

### SUMMARY

It is therefore an object of the invention to provide a sympathetic skin response measuring apparatus which can measure objectively and adequately a sympathetic skin response of the subject.

In order to achieve the object, according to the invention, there is provided a sympathetic skin response measuring apparatus comprising the features of claim 1.

### BRIEF DESCRIPTION OF THE DRAWINGS

Fig. 1 is a diagram showing an embodiment of the sympathetic skin response measuring apparatus of the invention.
Fig. 2 is a sectional view showing a stimulation electrode which is used in the embodiment of the sympathetic skin response measuring apparatus of the invention.
Fig. 3 is a perspective view showing a first modification of the stimulation electrode which is used in the embodiment of the sympathetic skin response measuring apparatus of the invention.
Fig. 4 is a perspective view showing a second modification of the stimulation electrode which is used in the embodiment of the sympathetic skin response measuring apparatus of the invention.
Figs. 5A and 5B are views showing measurement waveforms of SSRs measured by the embodiment of the sympathetic skin response measuring apparatus of the invention.

### DETAILED DESCRIPTION OF EMBODIMENTS

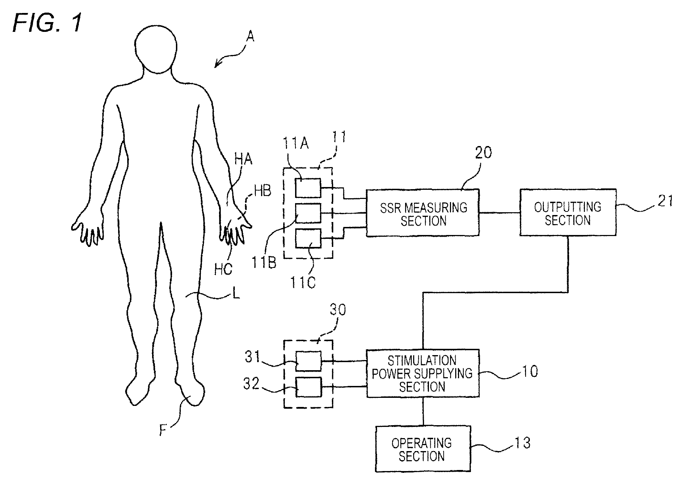

Hereinafter, an embodiment of the sympathetic skin response measuring apparatus of the invention will be described with reference to the accompanying drawings. In the figures, the identical components are denoted by the same reference numerals, and duplicate description is omitted. Fig. 1 shows the configuration of a sympathetic skin response measuring apparatus of the embodiment. The sympathetic skin response measuring apparatus includes a stimulation electrode 30 and measurement electrode 11 which are to be applied to the living body A. The measurement electrode 11 is configured by electrodes 11A, 11B, 11C.

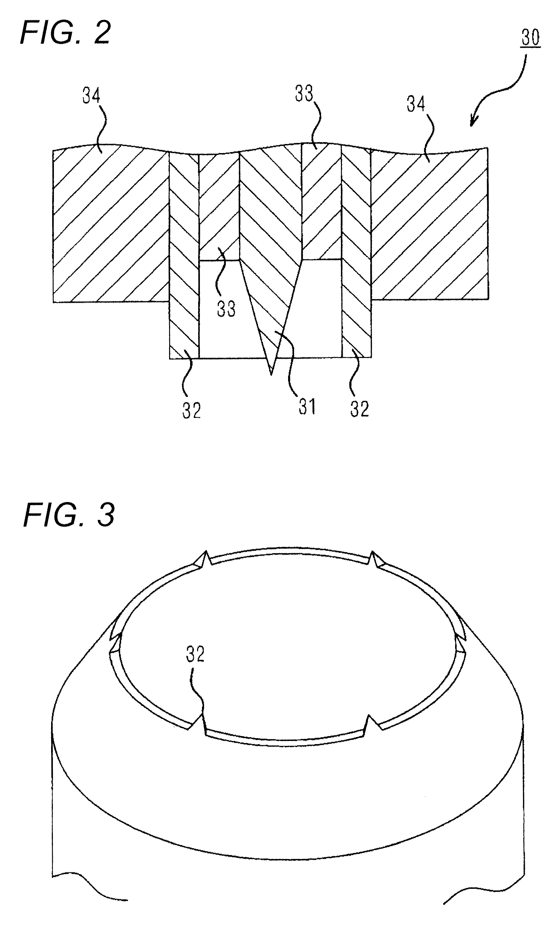

For example, the stimulation electrode is configured so as to have a section shown in Fig. 2. The stimulation electrode 30 includes a needle electrode 31 functioning as a first electrode in which the tip end has a shape that allows the tip end to be slightly inserted to the skin, and a contact electrode 32 functioning as a second electrode which is to be used while being in contact with the skin. When the needle electrode 31 is gently pressed against the skin, the tip end of the needle electrode 31 is inserted adjacent to the nerve endings of the myelinated fibres in the epidermis and superficial part of the dermis. As seen from Fig. 2, the needle electrode 31 is projected with respect to the contact electrode 32. The tip end of the needle electrode 31 is not always necessary to be pointed, and may have a spherical or rod-like shape. The contact electrode 32 may have a cylindrical shape which surrounds the needle electrode 31 while being centered at the needle electrode 31, or alternatively a plurality of contact electrodes 32 may be cylindrically placed so as to be centered at the needle electrode 31. The contact electrode has an inner diameter of, for example, 1 mm.

Alternatively, a part of the contact electrode 32 may have a shape which can be slightly inserted to the skin, as shown in Fig. 3. A spacer 33 configured by an insulating material may be embedded in the gap between the contact electrode 32 and the needle electrode 31. An external fitting portion 34 which has a columnar shape using the contact electrode 32 as a core, and which is formed by an insulating material is disposed in the circumference of the contact electrode 32.

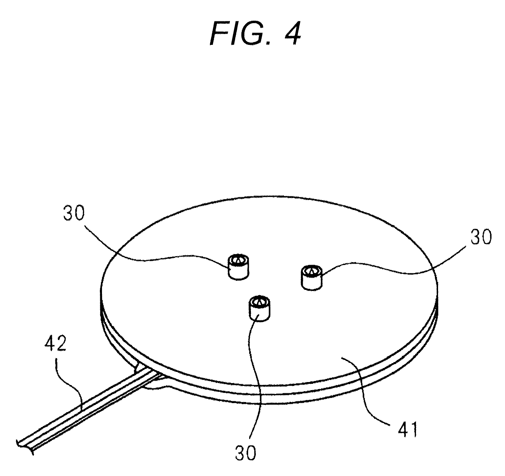

A configuration may be employed where, as shown in Fig. 4, a plurality (in this example, three pairs (poles)) of stimulation electrodes 30 each formed by a pair of the contact electrode 32 and needle electrode 31 which are indicated in Fig. 2 are used, the stimulation electrodes stand on a disk-like base 41 made of an insulating resin, and the three needle electrodes 31 are connected to one conductive wire, and the three contact electrodes 32 to one conductive wire to be led out as lead wires 42.

The stimulation electrode 30 is connected to a stimulation power supplying section 10. The stimulation power supplying section 10 supplies a power for stimulating only C fibers in the living body, and specifically performs a power supply while changing the electrical polarity of a pulse. The configuration of the stimulation power supplying section is similar to that disclosed in Japanese Patent Application No. 2008-264298 (The Japanese publication number is JP-A-2010-88802, the publication number of the corresponding US patent application is US2010/0094378A1 and the publication number of the corresponding EP patent application is EP2174589A1) which has been already filed by the present applicant. An operating section 13 configured by a pointing device, a keyboard, and the like is connected to the stimulation power supplying section 10. The stimulation intensity (mA), the rising/falling times of the pulse, the duration, interval, number, and shape of the pulse, the polarities of the electrodes, and the like can be set through the operating section 13. A displaying device which functions as an outputting section 21, and which is configured by, for example, an LCD is connected to the stimulation power supplying section 10. The contents which are set through the operating section 13 are displayed on the outputting section 21 which is the displaying device.

The electrodes 11A, 11B, 11C constituting the measurement electrode 11 are connected to an SSR measuring section 20. The SSR measuring section 20 measures information of a sympathetic skin response based on signals obtained from the measurement electrode 11. The SSR measuring section 20 detects the occurrence of sweating caused by excitation corresponding to stimulation, and specifically measures sweating by means of resistance changes between the electrodes 11A, 11B and the electrode 11C to obtain information of a sympathetic skin response. For example, the electrode 11A may be applied as a positive electrode to the left hand palm HC in which sweating easily occurs, the electrode 11B may be applied as a negative electrode to the left hand dorsum HB in which sweating hardly occurs, or which is free from sweating, and the electrode 11C may be applied as a ground electrode to the left wrist HA. On the other hand, the stimulation electrode 30 is applied to the left knee L and left foot instep F of the living body A so that stimulation can be applied. Measurement results are supplied to the outputting section 21 to be displayed thereon.

In an actual measurement, the three-pole electrode (surface stimulation electrode NM-980W manufactured by NIHON KOHDEN COOPERATION) shown in Fig. 4 was used as the stimulation electrode 30, portable peripheral nerve stimulation device PNS-7000 manufactured by the cooperation was used as the stimulation power supplying section 10, and electromyogram/evoked potential testing device MEB-2300 series Neuropack X1 manufactured by the cooperation was used as the SSR measuring section 20. Stimulation conditions were set as follows: rise (rising) = 0.3 ms, rise (falling) = 0.3 ms, pla (flat portion) = 0 ms, ISI (duration time) = 20 ms, train (pulse number) = 10, and the stimulation intensity was 0.10mA. A pulse signal in which the electrical polarity of the needle electrode 31 functioning as the first electrode is set to the positive pole, and that of the contact electrode 32 functioning as the second electrode is set to the negative pole is supplied by the stimulation power supplying section 10. Namely, the anode stimulation is performed. The SSR measurement was performed by repeating such stimulation three times with intervals of about one minute under the same stimulation conditions. At the same time, a response time measurement by using a push button was performed.

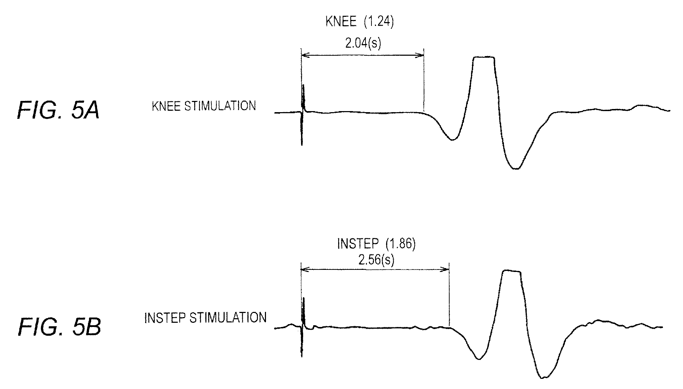

Fig. 5A shows a measurement waveform caused by a first knee stimulation, and Fig. 5B shows a measurement waveform caused by a first instep stimulation. During the measurement, the SSR measuring section 20 supplies the waveforms to the outputting section 21 so that they are displayed thereon, and obtains the SSR onset latency to be displayed. A configuration may be employed in which results of the response time measurement by using the push button are input through the operating section 13, and the SSR measuring section 20 obtains the contents of a comparison table of results such as shown in Table 1 below, and the time difference from stimulation, and supplies them to the outputting section 21 so that they are displayed thereon.

**TABLE 1**

| | SSR onset latency (s) | | Response time (s) | |
|---|---|---|---|---|
| | Instep | Knee | Instep | Knee |
| 1 | 2.56 | 2.04 | 1.86 | 1.24 |
| 2 | 2.48 | 2.16 | 1.52 | 1.54 |
| 3 | 2.56 | 2.00 | 1.54 | 1.12 |
| Average | 2.52 | 2.07 | 1.64 | 1.30 |
| Time difference | 0.45 | | 0.34 | |

| | | | | |
|---|---|---|---|---|
| Distance between the instep and the knee: 47 cm CV = 1.04 m/s | | | | |

Moreover, a configuration may be employed in which the distance between the foot instep and the knee is input through the operating section 13, and the SSR measuring section 20 obtains the conduction velocity of pain sensation through C fibers by using the time difference due to the average of the SSR offset latency, and supplies it to the outputting section 21 so that it is displayed thereon. When the results of Table 1 were used, conduction velocity CV = 1. 04 m/s, or namely a value which is close to well-known conduction velocity CV = 1 m/s of pain sensation through C fibers was obtained. It is known that, according to the sympathetic skin response measuring apparatus of the embodiment, only the C fibers among nerve fibers for skin sensation can be stimulated, and evaluation related to the sympathetic nerve can be performed objectively and adequately.

According to an aspect of invention, the stimulation power supplying section supplies a power for stimulating only C fibers in the living body, to the stimulation electrode. Therefore, only the C fibers among nerve fibers for skin sensation can be stimulated, and evaluation related to the sympathetic nerve can be performed objectively and adequately.

According to an aspect of the invention, the stimulation electrode is configured by the first electrode which is used while causing the tip end to be slightly inserted to the skin, and the second electrode which is placed in the circumference of the first electrode without electrical conduction therewith, and which is used while being in contact with the skin. The stimulation power supplying section supplies the pulse signal in which the electrical polarity of the first electrode is set to the positive pole, and that of the second electrode is set to the negative pole. Therefore, only the C fibers among nerve fibers for skin sensation can be stimulated, and evaluation related to the sympathetic nerve can be performed objectively and adequately.

According to an aspect of the invention, in the stimulation electrode, three pairs of the first electrode and the second electrode are disposed. Therefore, stable stimulation can be performed. Moreover, only the C fibers are simulated, and evaluation related to the sympathetic nerve can be performed objectively and adequately.

## Claims

1. A sympathetic skin response measuring apparatus comprising:
a stimulation electrode (30) and a measurement electrode (11) both adapted to be applied to a living body (A);
a stimulation power supplying section (10) configured to supply a power to the stimulation electrode (30);
a measuring section (20) configured to measure information of a sympathetic skin response based on a signal obtained from the measurement electrode (11); and
an outputting section (21) configured to output the information measured by the measuring section (20),
**characterized in that**
the stimulation electrode (30) includes: a first electrode (31) a tip end of which is adapted to be inserted to skin of the living body (A); and a second electrode (32) which is placed in a circumference of the first electrode (31) without electrical conduction with the first electrode (31) and which is adapted to be in contact with the skin, wherein the stimulation electrode (30) includes three pairs of the first electrode (31) and the second electrode (32); and
the stimulation power supplying section (10) is configured to supply a pulse signal, in which an electrical polarity of the first electrode (31) is set to a positive pole and an electrical polarity of the second electrode (32) is set to a negative pole, so as to supply power for stimulating only C fibers in a living body.

## Patentansprüche

1. Vorrichtung zum Messen der sympathischen Hautreaktion, die umfasst:
eine Stimulations-Elektrode (30) und eine Mess-Elektrode (11), die beide zum Anbringen an einem lebenden Körper (A) eingerichtet sind;
einen Teilabschnitt (10) zum Zuführen von Stimulations-Energie, der zum Zuführen einer Energie zu der Stimulations-Elektrode (30) eingerichtet ist;
einen Mess-Teilabschnitt (20), der zum Messen von Informationen einer sympathischen Hautreaktion auf Basis eines von der Mess-Elektrode (11) bezogenen Signals eingerichtet ist; und
einen Ausgabe-Teilabschnitt (21), der zum Ausgeben der durch den Mess-Teilabschnitt (20) gemessenen Informationen eingerichtet ist,
**dadurch gekennzeichnet, dass**
die Stimulations-Elektrode (30) eine erste Elektrode (31), deren vorderes Ende zum Einführen in Haut des lebenden Körpers (A) eingerichtet ist, sowie eine zweite Elektrode (32) enthält, die in einem Umkreis der ersten Elektrode (31) ohne elektrische Leitung mit der ersten Elektrode (31) positioniert ist und die so eingerichtet ist, dass sie in Kontakt mit der Haut ist, wobei die Stimulations-Elektrode (30) drei Paare aus der ersten Elektrode (31) und der zweiten Elektrode (32) enthält; und
der Teilabschnitt (10) zum Zuführen von Stimulations-Energie (10) so eingerichtet ist, dass er ein Impuls-Signal zuführt, wobei eine elektrische Polarität der ersten Elektrode (31) auf einen positiven Pol eingestellt ist und eine elektrische Polarität der zweiten Elektrode (32) auf einen negativen Pol eingestellt ist, um Energie zuzuführen, mit der nur C-Fasern in einem lebenden Körper stimuliert werden.

## Revendications

1. Appareil de mesure de la réponse sympathique de la peau comprenant :
une électrode de stimulation (30) et une électrode de mesure (11) conçues toutes les deux pour être appliquées sur un corps vivant (A),
une section d'alimentation en puissance de stimulation (10) configurée pour fournir de l'énergie à l'électrode de stimulation (30),
une section de mesure (20) configurée pour mesurer des informations sur la réponse sympathique de la peau fondées sur un signal obtenu à partir de l'électrode de mesure (11), et
une section de sortie (21) configurée pour délivrer en sortie les informations mesurées par la section de mesure (20),
**caractérisé en ce que**
l'électrode de stimulation (30) inclut : une première électrode (31) dont une extrémité en pointe est conçue pour être insérée sur la peau du corps vivant (A), ainsi qu'une seconde électrode (32) qui est placée à la circonférence de la première électrode (31), sans conduction électrique avec la première électrode (31), et qui est conçue pour être en contact avec la peau, l'électrode de stimulation (30) incluant trois paires constituées de la première électrode (31) et de la seconde électrode (32), et
la section d'alimentation en puissance de stimulation (10) est configurée pour fournir un signal impulsionnel dans lequel la polarité électrique de la première électrode (31) est fixée au pôle positif, et la polarité électrique de la seconde électrode (32) est fixée au pôle négatif, de façon à fournir de l'énergie pour ne stimuler que les fibres C dans un corps vivant.
